# FASCICULE DE BREVET EUROPEEN

(11) **EP 3 227 308 B1**
(45) Date de publication et mention de la délivrance du brevet: **10.10.2018**
(21) Numéro de dépôt: 15808823.7
(22) Date de dépôt: 03.12.2015
(51) Int. Cl.: C07H 1/00, C07H 15/04, C07D 307/20, C07C 41/09, B01F 17/42, C11D 1/66, C10M 105/18

(54) **SYNTHESE DE MONOÉTHERS DE SUCRE À LONGUE CHAINE ALKYLE ET LEURS UTILISATIONS COMME AGENT TENSIOACTIF**
SYNTHESE VON ZUCKERMONOÄTHER MIT LANGKETTIGER ALKYLGRUPPE UND IHRE VERWENDUNG ALS TENSIDE
SYNTHESIS OF SUGAR MONOETHERS WITH A LONG ALKYL CHAIN AND THEIR USE AS SURFACTANTS

(30) Priorité: 03.12.2014 FR 1402755
(43) Date de publication de la demande: 11.10.2017
(73) Titulaire: TEREOS STARCH & SWEETENERS BELGIUM, 9300 Aalst (BE); Université Claude Bernard Lyon 1, 69622 Villeurbanne Cedex (FR); Centre National de la Recherche Scientifique, 75016 Paris (FR)
(72) Inventeur: GOZLAN, Charlotte, 93190 LIVRY GARGAN (FR); DUCLOS, Marie-christine, F-69622 Villeurbanne Cedex (FR); DUGUET, Nicolas, F-69622 Villeurbanne Cedex (FR); LEMAIRE, Marc, F-69622 Villeurbanne Cedex (FR); REDL, Andreas, B-9300 Aalst (BE)
(74) Mandataire: Chielens, Kristof
(86) Numéro de dépôt international: PCT/IB2015/059328
(87) Numéro de publication internationale: WO 2016/088076

(56) Documents cités:
- EP-A1- 0 019 999
- WO-A1-2012/148530
- FANTON E ET AL: "Long-chain acetals derived from sucrose as a new class of surfactants", CARBOHYDRATE RESEARCH, PERGAMON, GB, vol. 298, no. 1-2, 20 février 1997 (1997-02-20), pages 85-92, XP004109759, ISSN: 0008-6215, DOI: 10.1016/S0008-6215(96)00300-X cité dans la demande

## Description

La présente invention concerne un procédé d'obtention d'un mélange de monoéthers d'alkyle en C4-C8 et en C9-C18 de saccharide ou d'un dérivé de saccharide ou de leurs mélanges.

L'invention porte en outre sur un mélange de monoéthers d'alkyle en C4-C8 et en C9-C18 de saccharide, d'un dérivé de saccharide ou de leurs mélanges, susceptible d'être obtenu par le dit procédé et son utilisation comme agent tensioactif.

### ARRIERE-PLAN DE L'INVENTION

Au cours du siècle dernier, on a observé une prise de conscience et une nette sensibilisation de notre société aux questions environnementales, et plus particulièrement à l'impact écologique de l'utilisation des produits chimiques dans l'industrie. Ceci a conduit à une augmentation de l'utilisation de produits biodégradables tels que par exemple, des agents tensioactifs biodégradables non toxiques. Les agents tensioactifs à base de sucres issus de ressources renouvelables représentent une bonne solution à ce besoin puisqu'ils possèdent de bonnes propriétés tensioactives. Leur utilisation en tant qu'émulsifiants dans l'industrie alimentaire et dans les réactions de polymérisation a été documentée que ce soit dans les détergents, les cosmétiques et les produits de nettoyage de surfaces. Deux grandes catégories d'agents tensioactifs à base de glucides sont commercialisés aujourd'hui: les esters d'acides gras à base de glucides, et les alkyles (poly) glycosides. Les esters d'acides gras à base d'hydrate de carbone ne sont stables que sur une gamme limitée de pH, car ils s'hydrolysent dans des conditions acides ou alcalines. Par contre, les alkyles (poly) glycosides sont très stables dans des conditions alcalines, mais ils sont sensibles en milieu acide. Les plus connus dans cette catégorie sont les alkylpolyglucosides (APG).

Dans la catégorie des esters d'acides gras à base de glucides, les esters d'acides gras et de sorbitol (SFAEs), de sorbitane (1,4-anhydrosorbitol), d'isosorbide (1,4-3,6-dianhydrosorbitol) ou de saccharose sont parmi les tensioactifs non-ioniques les plus facilement disponibles dans le commerce. Cependant, les agents tensioactifs ayant une fonction ester ne sont pas stables dans des conditions acides et basiques. Afin de remédier à ce problème, les inventeurs se sont proposés de remplacer la liaison ester par une fonctionnalité éther pour améliorer la résistance de ces espèces. Cependant, la préparation de dérivés d'éthers à base de glucides nécessite généralement l'utilisation de solvants chers et/ou toxiques (le diméthylsulfoxyde, le diméthylformamide, le diméthylacétamide), des températures élevées ou des étapes de protection/déprotection, l'utilisation de réactifs non-recyclables, de bases fortes (tels que l'hydroxyde de sodium ou de potassium ou l'hydrure de sodium), ou l'utilisation d'agents réducteurs qui produisent des déchets ou qui ont besoin d'être hydrolysés à la fin de la réaction (tels que les hydrures de sodium, d'aluminium ou de bore, les hydrosilanes et les hydrosiloxanes).

Dans ce contexte, les inventeurs ont récemment rapporté dans la demande de brevet français N°14 / 01346 un procédé de synthèse efficace des éthers de sucre par hydrogénolyse des acétals de sucre correspondants. En effet, les inventeurs ont mis au point un procédé de synthèse d'éthers de sucre, avec ou sans solvant par une première étape d'acétalisation ou de trans-acétalisation en utilisant un catalyseur et une seconde étape de réduction des acétals de sucre par hydrogénolyse pour obtenir un mélange de régio-isomères d'éthers de sucre. Ces deux étapes ont été optimisées sans purification des intermédiaires acétals afin de réduire le coût global du procédé.

Afin d'obtenir avec un bon rendement, une composition ayant des propriétés tensioactives élevées et stables notamment par la mise en oeuvre d'un procédé qui respecte les contraintes industrielles, économiques et environnementales, les inventeurs ont émis l'hypothèse que ce procédé pouvait être mis en oeuvre pour l'obtention d'une composition d'éthers de sucres à longues chaînes.

Cependant, les inventeurs ont constaté que ce procédé n'est pas adapté à une étape d'acétalisation utilisant un aldéhyde à longue chaîne alkyle. En effet, en utilisant des réactifs à longue chaîne alkyle, de faibles rendements sont obtenus.

Il existe donc un besoin d'un procédé économique et respectueux de l'environnement pour la production d'une composition écologique ayant des propriétés tensioactives élevées et stables dans le temps et vis-à-vis des variations de pH.

A la suite de nombreux travaux expérimentaux, les inventeurs ont réussi à apporter une solution aux problèmes mentionnés ci-dessus. Ainsi, les inventeurs ont mis en place pour la première fois, une nouvelle voie pour la synthèse de monoéthers d'alkyle à chaînes longues de saccharides ayant des caractéristiques de tensioactifs. En effet, les inventeurs ont montré qu'un meilleur rendement de la synthèse d'acétal d'alkyle à chaîne longue de saccharide peut être obtenu en utilisant un aldéhyde d'alkyle à chaîne courte comme co-réactif. Les inventeurs ont mis en place un procédé efficace pour la synthèse de monoéthers d'alkyle à chaînes longues de saccharides ayant des caractéristiques de tensioactifs en utilisant un agent réducteur qui ne produit pas de déchets (tel que l'hydrogène), à la fin de la réaction comparativement à d'autres méthodes de synthèse d'éther utilisant des hydrures en tant qu'agent réducteur. Le procédé de synthèse de monoéthers d'alkyle à chaînes longues de saccharides tel que proposé par les inventeurs peut être mis en oeuvre en utilisant uniquement des réactifs biosourcés qui sont ou peuvent être synthétisés à partir de matières premières renouvelables (tels que des aldéhydes gras qui peuvent être préparés à partir d'acides gras ou les saccharides ou les polyols pouvant être obtenus à partir d'amidon). Les inventeurs ont également mis au point un procédé de synthèse de monoéthers d'alkyle à chaînes longues de saccharides comprenant une séquence réactionnelle impliquant deux réactions d'acétalisation et d'une réaction d'hydrogénolyse, sans la nécessité d'une étape de purification du mélange brut d'acétals de sucre. Enfin, les inventeurs ont mis au point un moyen économique, efficace et respectueux de l'environnement pour synthétiser des compositions de monoéthers d'alkyle de saccharides comprenant des chaînes longues ou des chaînes courtes, ces compositions d'éthers de saccharides montrant des propriétés de tension de surface améliorées et des propriétés tensioactives similaires à celles des tensioactifs à chaîne alkyle longue seuls. Les inventeurs ont mis en évidence un effet synergique entre les tensioactifs à chaînes alkyles courtes et longues permettant à la fois une diminution de la tension superficielle de l'eau et une augmentation de la solubilité du tensioactif à chaîne alkyle longue en solution aqueuse.

EP 0 019 999 A1 décrit la préparation d'acetals alkyles à longues chaînes de saccharide, en particulier de sorbitol partiellement substitué de groupes d'acétate à partir d'aldéhydes en C₇-C₃₀ comprenant préférentiellement un mélange de chaînes alkyles C₁₂/C₁₄ 70 : 30 et d'acide acétique en tant que milieu réactionnel.

Fanton E et Al : « Long-chain acetals derived from sucrose as a new class of surfactants », Carbohydrate research, pergamon, GB, vol. 298, no. 1-2, 20 février 1997, pages 85-92, décrit la préparation d'acétals alkyles supérieurs en C₆-C₁₈ de sucrose acétylé à partir des aldéhydes correspondants.

WO 2012/148530 décrit des compositions d'éthers monoalkyliques de polyols et plus particulièrement de mono-anhydro hexitol présentant un radical éther alkyle en C₄-C₁₈.

### DESCRIPTION DE L'INVENTION

### Procédé d'obtention d'un mélange de monoéthers d'alkyle de saccharide ou d'une composition comprenant un monoéther d'alkyle en C9-C18 de saccharide

L'invention concerne un procédé d'obtention d'un mélange de monoéther d'alkyle en C4-C8 de saccharide et/ou de dérivé de saccharide et de monoéther d'alkyle en C9-C18 de saccharide et/ou de dérivé de saccharide, ledit dérivé de saccharide étant un saccharide alkylé et/ou hydrogéné et/ou déshydraté, le procédé comprenant:
a) une première étape d'acétalisation ou de trans-acétalisation d'un saccharide, d'un dérivé de saccharide ou de leurs mélanges par un aldéhyde aliphatique en C4-C8 ou l'acétal de celui-ci,
b) une deuxième étape consécutive ou simultanée d'acétalisation ou de trans-acétalisation du produit obtenu en a), du saccharide, du dérivé de saccharide ou de leurs mélanges par un aldéhyde aliphatique en C9-C18 ou l'acétal de celui-ci,
c) une étape d'hydrogénolyse catalytique des acétals de saccharide et/ou de dérivé de saccharide obtenus en b), et
d) une étape de récupération d'un mélange de monoéther d'alkyle en C4-C8 de saccharide et/ou de dérivé de saccharide et de monoéther d'alkyle en C9-C18 de saccharide et/ou de dérivé de saccharide.

L'invention concerne également un procédé d'obtention d'une composition comprenant un monoéther d'alkyle en C9-C18 de saccharide et/ou de dérivé de saccharide, ledit dérivé de saccharide étant un saccharide glycosylé et/ou hydrogéné et/ou déshydraté, le procédé comprenant:
a) une première étape d'acétalisation ou de trans-acétalisation d'un saccharide, d'un dérivé de saccharide ou de leurs mélanges par un aldéhyde aliphatique en C4-C8 ou l'acétal de celui-ci,
b) une deuxième étape d'acétalisation ou de trans-acétalisation du produit obtenu en a), du saccharide, du dérivé de saccharide ou de leurs mélanges par un aldéhyde aliphatique en C9-C18 ou l'acétal de celui-ci, ladite étape b) pouvant être consécutive ou simultanée à l'étape a)
c) une étape d'hydrogénolyse catalytique des acétals de saccharide et/ou de dérivé de saccharide obtenus en b), et
d) une étape de récupération d'une composition comprenant un monoéther d'alkyle en C9-C18 de saccharide et/ou de dérivé de saccharide.

On entend par « acétalisation » la réaction par laquelle un groupement alcool et un carbonyle forment un groupement acétal. Cette réaction est largement décrite dans l'art antérieur et bien connue de l'homme du métier. On entend par « trans-acétalisation » la réaction par laquelle un transfert de groupements acétals est opéré entre un composé carbonylé et un autre. Cette réaction est également bien connue de l'homme du métier.

Tel qu'utilisé ici, le terme "saccharide" se réfère à n'importe quel hydrate de carbone simple, y compris les monosaccharides ou des oligosaccharides substitués et non substitués. En règle générale, des exemples de saccharides peuvent être des monosaccharides, des disaccharides ou des trisaccharides. Des exemples de mélange de saccharides peuvent être un mélange de monosaccharides et de disaccharides, ou un mélange de monosaccharides et de trisaccharides ou de disaccharides et de trisaccharides.

Tel qu'utilisé ici, le terme «monosaccharide» se réfère au polyhydroxyaldehyde (aldose) ou au polyhydroxyketone (cétose) et leurs dérivés ou leurs analogues. Selon l'invention, ledit monosaccharide peut comporter de 4 à 7 atomes de carbone. Des exemples de monosaccharides convenables comprennent le ribose, le xylose, l'arabinose, le glucose, le galactose, le mannose, le telose, le gulose, l'allose, l'altrose, l'idose, le lyxose, le ribulose, le sorbose, le fructose, et leurs mélanges.

De préférence, ladite unité de monosaccharide présente 6 atomes de carbone, également désignée sous le nom d'« hexose ». Le terme « hexose » se réfère à la fois aux aldohexoses, aux cétohexoses, à leurs dérivés et analogues.

De préférence, ledit hexose est choisi dans le groupe constitué par le glucose, le mannose, le galactose, l'allose, l'altrose, le gulose, l'idose et le talose.

Selon un mode de réalisation, préalablement aux étapes a) et b) d'acétalisation ou de trans-acétalisation, le saccharide est alkylé, hydrogéné et/ou déshydraté afin d'obtenir un un alkylglycoside, un sucre-alcool ou un anhydrosaccharide.

Selon un mode de réalisation, l'étape a) du procédé selon l'invention peut être précédée d'une étape d'hydrogénation dudit saccharide ou peut être réalisée à partir d'un dérivé de saccharide hydrogéné également appelé un sucre-alcool. Tel qu'il est utilisé ici, le terme « sucre-alcool », également connu sous le nom «polyol» se réfère à une forme hydrogénée de saccharide, tels que les mono, di ou tri-saccharides, dont le groupe carbonyle (aldéhyde ou cétone) a été réduit en un hydroxyle primaire ou secondaire. Ledit sucre-alcool peut être, par exemple, choisi dans le groupe constitué par l'érythritol, le thréitol, l'arabitol, le xylitol, le ribitol, le mannitol, le sorbitol, le galactitol, l'iditol, le volémitol, l'isomalt, le maltitol, le lactitol, le maltotriitol, le maltotétraitol et le polyglycitol. De préférence, le sucre-alcool est le sorbitol, le xylitol ou le mannitol.

Typiquement, ledit sucre-alcool est un pentitol choisi dans le groupe constitué par le xylitol, l'arabitol et le ribitol, ou un hexitol choisi dans un groupe constitué par le mannitol, le sorbitol, le galactitol, le fucitol, l'iditol et l'inositol.

Typiquement, l'étape a) du procédé selon l'invention peut être précédée d'une étape d'hydrogénation puis de déshydratation dudit saccharide ou peut être réalisée à partir d'un dérivé de saccharide hydrogéné puis déshydraté également appelé un anhydrosaccharide.

Un « anhydrosaccharide » doit être compris comme étant un saccharide obtenu par déshydratation, par l'élimination d'une ou plusieurs molécules d'eau à partir d'un mono-, di-, tri- ou oligo-saccharide hydrogéné correspondant. Un exemple d'anhydrosaccharide approprié peut être un monoanhydrosaccharide tel qu'un hexitan choisi parmi le groupe constitué par le 1,4-anhydro-D-sorbitol (1,4-arlitan ou sorbitan); 1,5-anhydro-D-sorbitol (polygalitol); 3,6-anhydro-D-sorbitol (3,6-sorbitan); 1,4 (3,6) -anhydro-D-mannitol (mannitan); 1,5-anhydro-D-mannitol (styracitol); 3,6-anhydro-D-galactitol; 1,5-anhydro-D-galactitol; 1,5-anhydro-D-talitol et 2,5-anhydro-L-iditol.

L'hexitan préféré est dérivé de la déshydratation du sorbitol pour former par exemple, le 1,4-sorbitane, le 3,6-sorbitane ou le 2,5-sorbitane.

Selon un mode de réalisation, le procédé selon l'invention comprend une étape de déshydratation à la suite d'une étape d'hydrogénation d'un saccharide ou à partir d'un dérivé de saccharide tel qu'un sucre-alcool. Typiquement, une telle étape de déshydratation est effectuée avant ladite étape a) de première acétalisation ou trans-acétalisation afin d'obtenir un anhydrosaccharide. Lorsqu'elle est effectuée sur un dérivé de saccharide, tel qu'un sucre-alcool, le dérivé de saccharide préférentiellement sous forme pulvérulente est fondu avant l'étape de déshydratation. L'étape de déshydratation peut être effectuée avec un catalyseur par exemple, avec un catalyseur acide.

Selon l'invention, l'étape de déshydratation est effectuée sous atmosphère d'hydrogène à une pression de préférence d'environ de 20 à 50 bar.

Avantageusement, l'étape de déshydratation est effectuée à une température comprise entre 120 et 170°C, de préférence entre 130 et 140°C.

Typiquement, le saccharide est purifié après l'étape de déshydratation, par exemple par cristallisation, recristallisation ou chromatographie.

Avantageusement, le dérivé de saccharide est un saccharide alkylé. Il peut être trouvé dans le commerce ou être obtenu par glycosylation d'un saccharide avant ladite étape a) de première acétalisation ou trans-acétalisation afin d'obtenir un alkylglycoside.

Tel qu'utilisé ici, le terme "alkylglycoside" se réfère à n'importe quel saccharide notamment, un monosaccharide, un disaccharide ou un trisaccharide relié par une liaison à un groupe alkyl, tel que décrit dans l'état de la technique. Typiquement, le saccharide peut être lié au groupement alkyle par un atome d'oxygène (un O-glycoside), un atome d'azote (une glycosylamine), un atome de soufre (un thioglycoside), ou un atome de carbone (un C-glycoside). Le groupe alkyle peut avoir une longueur de chaîne variée de préférence, le groupe alkyle est un groupe alkyle en C1-C4. Un groupe alkyle encore plus préféré est un méthyle ou un éthyle. Des alkyles glycosides peuvent être par exemple choisis parmi un groupe constitué du glucoside de méthyle, du glucoside d'éthyle, du glucoside de propyle, du glucoside de butyle, du xyloside de méthyle, du xyloside d'éthyle, du xyloside de propyle, du xyloside de butyle, du mannoside de méthyle, du mannoside d'éthyle, du mannoside de propyle, du mannoside de butyle, du galactoside de méthyle, du galactoside d'éthyle, du galactoside de propyle et du galactoside de butyle.

Par « leurs mélanges », on entend un mélange de plusieurs saccharides, un mélange de plusieurs dérivés de saccharides ou un mélange d'un ou plusieurs saccharides avec un ou plusieurs dérivés de saccharides.

Selon l'invention, la première et/ou la deuxième étape d'acétalisation ou de trans-acétalisation comprend:
i) éventuellement, une étape de préchauffage dudit saccharide ou dudit mélange de saccharides, de préférence à une température comprise entre 70 et 130°C, typiquement entre 90 et 110°C,
ii) une étape d'addition de l'aldéhyde aliphatique ou d'un dérivé d'aldéhyde aliphatique audit saccharide ou audit mélange de saccharides, et
iii) une étape d'addition d'un catalyseur de préférence d'un catalyseur acide.

L'étape i) est particulièrement avantageuse en ce qu'elle peut être mise en oeuvre en l'absence de solvant.

De préférence, le catalyseur acide utilisé dans la première et/ou la deuxième étape d'acétalisation ou de trans-acétalisation et le cas échéant lors de l'étape de déshydratation peut être un catalyseur acide homogène ou hétérogène. Le terme «homogène», tel qu'utilisé dans l'expression « catalyseur acide homogène » se réfère à un catalyseur qui se trouve dans la même phase (solide, liquide ou gaz) ou dans le même état d'agrégat que le réactif. Inversement, le terme « hétérogène », tel qu'utilisé dans l'expression « catalyseur acide hétérogène » se réfère à un catalyseur qui se trouve dans une phase différente (solide, liquide ou gaz) que les réactifs.

Ledit catalyseur acide utilisé dans la première et/ou la deuxième étape d'acétalisation ou de trans-acétalisation et le cas échéant lors de l'étape de déshydratation peut être indépendamment choisi parmi les acides solides ou liquides, organiques ou inorganiques, les acides solides étant préférés. En particulier, le catalyseur acide préféré est choisi parmi l'acide para-toluène sulfonique, l'acide méthane sulfonique, l'acide camphosulfonique (CSA) et les résines sulfoniques.

Typiquement, la première et/ou la deuxième étape d'acétalisation ou de trans-acétalisation est effectuée à des températures comprises entre 70 et 130°C, typiquement entre 70 et 90°C. La température des mélanges réactionnels peut varier en fonction des réactifs et solvants utilisés. Le temps de réaction est déterminé par le degré de conversion atteint.

Selon un mode de réalisation, la première et/ou la deuxième acétalisation ou trans-acétalisation peut être effectuée de manière indépendante par un aldéhyde aliphatique ou l'acétal de celui-ci, typiquement, un aldéhyde aliphatique linéaire ou ramifié ou l'acétal de celui-ci. La première étape a) d'acétalisation ou de trans-acétalisation peut être réalisée typiquement avec un aldéhyde aliphatique ou l'acétal de celui-ci ayant 4, 5, 6, 7 ou 8 atomes de carbone, par exemple choisi parmi le butanal, le pentanal, l'hexanal, l'heptanal, l'octanal et leurs acétals. De préférence, l'aldéhyde aliphatique en C4-C8 est un aldéhyde aliphatique en C5 ou l'acétal de celui-ci par exemple, un pentanal ou l'acétal de celui-ci. La deuxième étape b) d'acétalisation ou de trans-acétalisation peut être typiquement réalisée avec un aldéhyde aliphatique ou l'acétal de celui-ci ayant 9, 10, 11, 12, 13, 14, 15, 16, 17 ou 18 atomes de carbone, par exemple choisi parmi le nonanal, décanal, le undécanal, le dodécanal, le tridécanal, le tétradécanal, le pentadecanal, l'hexadécanal, l'heptadecanal, l'octodécanal et l'acétal. De préférence, l'aldéhyde aliphatique en C9-C18 ou l'acétal de celui-ci est un aldéhyde aliphatique en C12 ou l'acétal de celui-ci par exemple, un dodécanal ou l'acétal de celui-ci.

L'expression « l'acétal de celui-ci » ou « leur(s) acétal(s) », telle qu'utilisée par exemple dans l'expression « aldéhyde aliphatique en C4-C8 ou l'acétal de celui-ci » ou «aldéhyde aliphatique en C9-C18 ou l'acétal de celui-ci» englobe le di-alkyl acétal de l'aldéhyde aliphatique en C4-C8 ou en C9-C18 correspondant. Plus particulièrement, les acétals de di-méthyle ou de di-éthyle de l'aldéhyde aliphatique en C4-C8 ou en C9-C18 sont préférés.

Selon un mode de réalisation, la première étape a) et/ou la deuxième étape b) d'acétalisation ou de trans-acétalisation peut être effectuée indépendamment l'une de l'autre avec ou sans solvant. Lorsque la réaction est effectuée en présence d'un solvant, le solvant est de préférence un solvant polaire.

Typiquement, le solvant peut être choisi parmi le diméthylformamide (DMF), le diméthylsulfoxyde (DMSO), le diméthylacétamide (DMA), l'acétonitrile (CH₃CN), le tétrahydrofurane (THF), le 2-méthyltétrahydrofuranne (2Me-THF), l'éther méthylique de cyclopentyle (CPME), le methanol (MeOH), l'éthanol (EtOH), le propanol (PrOH), l'isopropanol (iPrOH), le butanol (BuOH), l'éther dibutylique (DBE), le méthyle tert-butyle éther (MTBE) et le triméthoxypropane (TMP).

Des travaux expérimentaux approfondis ont conduit à une sélection de conditions permettant l'observation de taux de conversion et de rendements optimaux au cours des étapes d'acétalisation ou de trans-acétalisation. Les meilleurs résultats ont été obtenus lorsque le rapport molaire [(aldéhyde aliphatique en C4-C8 ou en C9-C18 ou leur acétal) : (saccharide, dérivé de saccharide ou leurs mélanges)] est compris entre 5:1 et 1:5, de préférence entre 4:1 et 1:4, et de manière avantageuse entre 3:1 et 1:3. L'expression "rapport molaire (aldéhyde aliphatique en C4-C8 ou en C9-C18 ou leur acétal) : (saccharide, dérivé de saccharide ou leurs mélanges) " désigne le rapport molaire de l'aldéhyde aliphatique en C4-C8: (saccharide, dérivé de saccharide ou leurs mélanges) ou le rapport molaire de l'aldéhyde aliphatique en C9-C18 : (saccharide, dérivé de saccharide ou leurs mélanges) mais également, le rapport molaire de l'acétal aliphatique en C4-C8 : (saccharide, dérivé de saccharide ou leurs mélanges) ou le rapport molaire de l'acétal aliphatique en C9-C18 : (saccharide, dérivé de saccharide ou leurs mélanges).

Les inventeurs ont montré plus particulièrement, que lors d'une réaction d'acétalisation, le rapport molaire aldéhyde aliphatique en C4-C8 ou en C9-C18 : (saccharide, dérivé de saccharide ou leurs mélanges) compris entre 1:1 et 1:5 , de préférence entre 1:1 et 1:4, et de manière préférée entre 1:3 et 1:2 améliore les rendements et fournit des taux optimaux de conversion.

Les inventeurs ont montré en outre, qu'au cours des réactions de trans-acétalisation, un rapport molaire acétal aliphatique en C4-C8 ou en C9-C18 : (saccharide, dérivé de saccharide ou leurs mélanges) compris entre 1:1 et 5:1, de préférence entre 5:4 et 4:1, de préférence entre 3:1 et 4:3, de préférence encore entre 3:2 et 2:5 améliore les rendements et fournit des taux optimaux de conversion. Les catalyseurs utilisés sont les mêmes que lors de la réaction d'acétalisation.

Selon un mode de réalisation, le procédé de l'invention comprend en outre, au moins une étape de neutralisation et/ou de filtration et/ou de purification après l'une quelconque des étapes de déshydratation le cas échant, de première étape a) et/ou de deuxième étape b) d'acétalisation ou de trans-acétalisation.

Lorsqu'une étape de purification est prévue, ladite étape de purification peut être par exemple, une cristallisation, une recristallisation ou une chromatographie. De préférence, la chromatographie est réalisée en utilisant un solvant polaire non-aqueux. En général, quand une étape de filtration et/ou de purification est prévue avant l'étape d'hydrogénolyse, le solvant polaire non-aqueux peut être identique à celui utilisé lors de l'étape d'hydrogénolyse.

Avantageusement, l'étape d'hydrogénolyse est effectuée à une température comprise entre 80°C et 140°C, et/ou à une pression d'hydrogène comprise entre 15 et 50 bar, de préférence entre 20 et 40 bar.

L'étape d'hydrogénolyse est effectuée avantageusement dans un solvant polaire aprotique, de préférence un solvant non-aqueux. En effet, les solvants aprotiques offrent une meilleure conversion. Des exemples de solvants aprotiques sont, entre autres, sans limitation, les alcanes, le 1,2,3-triméthoxypropane (TMP), le tert-butyle éther de méthyle (MTBE), le tétrahydrofurane (THF), le 2-méthyl tétrahydrofuranne (2Me-THF), l'éther de dibutyle (DBE) et le cyclopentylméthyléther (CPME). De préférence, le solvant aprotique est le CPME. Les alcanes sont avantageux car ils permettent une meilleure solubilisation de l'hydrogène dans le milieu. Cependant, la conversion est moins élevée que d'autres solvants aprotiques tels que le CPME. En général, parmi les alcanes, le dodécane et l'heptane sont préférés.

L'étape d'hydrogénolyse est effectuée de préférence dans un solvant aprotique polaire à une température comprise entre 80°C et 140°C et/ou sous une pression d'hydrogène comprise entre 15 et 50 bar, en présence d'un catalyseur adapté aux réactions d'hydrogénolyse.

De préférence, l'étape d'hydrogénolyse est effectuée dans un solvant polaire non-aqueux à une température comprise entre 100°C et 130°C et/ou à une pression comprise entre 25 et 35 bar.

Généralement, l'hydrogénolyse est effectuée en présence d'un catalyseur adapté tel qu'un catalyseur à base de métaux précieux ou de métaux communs. Plus particulièrement, les métaux communs peuvent être des métaux ferreux ou non-ferreux. Typiquement, l'hydrogénolyse est effectuée en présence d'un catalyseur à base de métaux ferreux.

A titre indicatif, un catalyseur de métal appartenant au groupe des métaux ferreux peut être du nickel, du cobalt ou du fer.

De préférence, l'hydrogénolyse est réalisée en utilisant un catalyseur à base de métaux précieux tels que le palladium, le rhodium, le ruthénium, le platine ou l'iridium.

En règle générale, le catalyseur utilisé au cours de l'hydrogénolyse peut être fixé sur un support tel que le carbone, l'alumine, la zircone ou la silice ou un mélange quelconque de ceux-ci. Un tel support est par exemple une bille. Ainsi, un catalyseur de palladium fixé sur des billes de charbon (Pd / C) peut être avantageusement utilisé. Ces catalyseurs peuvent être dopés par l'adjonction de métaux précieux ou métaux communs. On parle d'agent de dopage. Typiquement, l'agent de dopage représente 1 à 10% en poids du catalyseur.

### Mélange de monoéthers d'alkyle de saccharides

L'invention porte également sur une composition comprenant un mélange d'isomères de position de monoéther d'alkyle en C4-C8 de saccharide et/ou de dérivé de saccharide et d'isomères de position de monoéther d'alkyle en C9-C18 de saccharide et/ou de dérivé de saccharide, notamment, susceptible d'être obtenus par le procédé de l'invention, dans lequel le dérivé de saccharide est un saccharide alkylé et/ou hydrogéné et/ou déshydraté, et le saccharide est un hexose. Préférentiellement, les isomères de position de monoéther d'alkyle en C4-C8 de saccharide ou de dérivé de saccharide et les isomères de position de monoéther d'alkyle en C9-C18 de saccharide ou de dérivé de saccharide présentent des groupements alkyl en au moins deux des positions choisies parmi 2-O, 3-O, 4-O, 5-O et 6-O.

Typiquement, lorsque le dérivé de saccharide est un alkylglycoside les isomères de position sont au moins deux des 2-O monoether d'alkyle, 3-O monoether d'alkyle, 4-O monoether d'alkyle et 6-O monoether d'alkyle en C9-C18 ou en C4-C8.

Typiquement, lorsque le dérivé de saccharide est un hexitan, les isomères de position sont au moins deux des 2-O monoether d'alkyle, 3-O monoether d'alkyle, 5-O monoether d'alkyle et 6-O monoether d'alkyle en C9-C18 ou en C4-C8

Avantageusement, le groupement alkyle en C4-C8 est un alkyle en C5 et le groupement alkyle en C9-C18 est un alkyle en C12. Préférentiellement, le dérivé de saccharide est choisi parmi le monoanhydro sorbitol ou le glucoside d'alkyle encore plus préférentiellement, le dérivé de saccharide est le glucoside de méthyle.

L'invention porte également sur un mélange de monoéther d'alkyle en C4-C8 de dérivé de saccharide et de monoéther d'alkyle en C9-C18 de dérivé de saccharide, notamment, susceptible d'être obtenu par le procédé de l'invention, dans lequel le monoéther d'alkyle en C4-C8 de dérivé de saccharide est un monoéther d'alkyle en C5 de dérivé de saccharide et le monoéther d'alkyle en C9-C18 de dérivé de saccharide est un monoéther d'alkyle en C12 de dérivé de saccharide, de préférence, dans lequel ledit dérivé de saccharide est un saccharide glycosylé et/ou hydrogéné et/ou déshydraté plus préférentiellement ledit dérivé de saccharide est choisi parmi le monoanhydro sorbitol ou le glucoside d'alkyle notamment le méthyle glucoside.

En général, le rapport monoéther d'alkyle en C5 de saccharide / monoéther d'alkyle en C12 de saccharide dans le mélange de l'invention est compris entre 5:95 et 95:5, de préférence entre 20:80 et 70:30, 30:70 et 60:40, 35:65 et 55:45, 40:60 et 52:48, 42:58 et 50:50.

### Utilisation de monoéther d'alkyle de saccharide comme un agent tensioactif et méthode d'obtention d'un tel agent tensioactif

L'invention porte également sur l'utilisation du mélange obtenu par la mise en oeuvre du procédé selon l'invention ou l'utilisation d'un mélange de monoéther d'alkyle en C4-C8 de saccharide ou de dérivé de saccharide et de monoéther d'alkyle en C9-C18 de saccharide ou de dérivé de saccharide en tant qu'agent tensioactif, ledit dérivé de saccharide étant un saccharide glycosylé et/ou hydrogéné et/ou déshydraté. Préférentiellement, ledit dérivé de saccharide est choisi parmi le monoanhydro sorbitol ou le glucoside d'alkyle notamment le méthyle glucoside

Le terme « tensioactif » ou « agent tensioactif » également connu sous le nom « surfactant » désigne un composé qui réduit la tension superficielle lorsqu'il est dissout dans l'eau ou dans un milieu aqueux, ou qui réduit la tension interfaciale entre deux liquides, entre un liquide et un solide ou entre un liquide et un gaz. En règle générale, les propriétés tensioactives d'un composé peuvent être mesurées par la méthode de la plaque à l'aide d'une tige de platine en tant que sonde (Méthode Du Nouy-Padday telle que décrite dans JF Padday, AR Pitt, RMPashley, J. Chem. Soc. Faraday. Trans 1, 1975, 71, 1919-1931). La méthode Du Nouy-Padday est utilisée pour mesurer l'équilibre de tension superficielle ou de tension de surface dynamique à l'interface air-liquide.

Selon un mode de réalisation, le mélange obtenu par la mise en oeuvre du procédé selon l'invention ou le mélange de monoéther d'alkyle en C4-C8 de saccharide et de monoéther d'alkyle en C9-C18 de saccharide peut être utilisé dans une composition contenant au moins deux agents tensioactifs ou en tant que seul agent tensioactif.

Dans un mode de réalisation, le mélange obtenu selon le procédé de l'invention ou le mélange de monoéther d'alkyle en C4-C8 de saccharide et de monoéther d'alkyle en C9-C18 de saccharide peut être utilisé en tant que détergent, émulsifiant, stabilisateur d'émulsion, agent moussant, stabilisateur de mousse, stabilisateur de liposome, dispersant et/ou agent mouillant. De tels agents sont habituellement utilisés dans diverses applications domestiques et industrielles, comme par exemple comme détergent pour le lavage du linge, détergent pour laver la vaisselle, détergent industriel, émulsifiant dans les cosmétiques, émulsifiant et/ou stabilisant dans les encres, dans les peintures et dans les compositions de revêtement, et agent moussant et/ou stabilisant de mousse dans le shampoing.

Typiquement, le mélange de monoéther d'alkyle en C4-C8 de saccharide ou de dérivé de saccharide et de monoéther d'alkyle en C9-C18 de saccharide ou de dérivé de saccharide est un mélange de monoéther d'alkyle en C5 de saccharide ou de dérivé de saccharide et de monoéther d'alkyle en C12 de saccharide ou de dérivé de saccharide, ledit dérivé de saccharide étant un saccharide alkylé et/ou hydrogéné et/ou déshydraté de préférence, ledit dérivé de saccharide est un monoanhydrosorbitol ou un glucoside d'alkyle notamment un glucoside de méthyle.

L'invention porte également sur une méthode d'obtention d'un agent tensioactif ou d'une composition tensioactive comprenant
a) une première étape d'acétalisation ou de trans-acétalisation d'un saccharide, d'un dérivé de saccharide ou de leurs mélanges par un aldéhyde aliphatique en C4-C8 ou l'acétal de celui-ci, ledit dérivé de saccharide étant un saccharide glycosylé et/ou hydrogéné et/ou déshydraté
b) une deuxième étape consécutive ou simultanée d'acétalisation ou de trans-acétalisation du produit obtenu en a), du saccharide, du dérivé de saccharide ou de leurs mélanges par un aldéhyde aliphatique en C9-C18 ou l'acétal de celui-ci,
c) une étape d'hydrogénolyse catalytique des acétals de saccharide obtenus en b), et
d) une étape de récupération de l'agent tensioactif ou de la composition tensioactive obtenu(e).

Typiquement, ladite composition tensioactive est un détergent, un émulsifiant, un stabilisateur d'émulsion, un agent moussant, un stabilisateur de mousse, un stabilisateur de liposome, un dispersant et/ou un agent mouillant.

Bien qu'ayant des significations distinctes, les termes « comprenant », « contenant », « comportant » et « consistant en » ont été utilisés de manière interchangeable dans la description de l'invention, et peuvent être remplacés l'un par l'autre.

L'invention sera mieux comprise à la lecture des figures et exemples suivants donnés uniquement à titre d'exemple.

### BREVE DESCRIPTION DES FIGURES

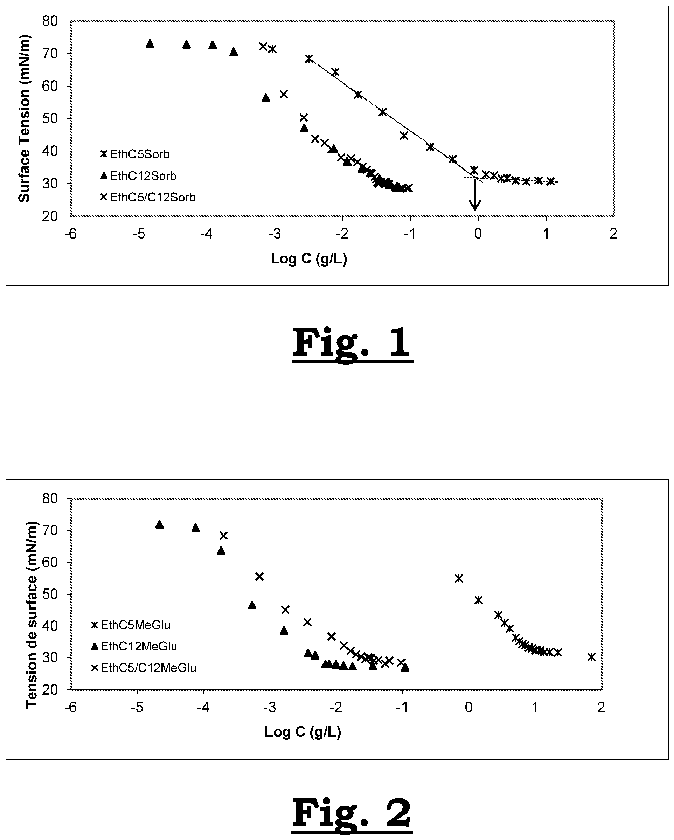

- La figure 1 représente la tension de surface (mN/m) des dérivés d'éthers de sorbitane en fonction de la concentration (g/L). La concentration micellaire critique (CMC) est indiquée par de petites flèches, le C5EthSorb est représenté par les losanges noirs, le C12EthSorb par les points noirs et le mélange C5 / C12EthSorb par les triangles vides.
- La figure 2 représente la tension superficielle (mN/m) des dérivés d'éther de glucoside de méthyle en fonction de la concentration (g/L). Le C5EthMeGlu est représenté par les étoiles noires, le C12EthMeGlu par les losanges noirs, et le mélange C5 / C12EthMeGlu par les croix noires.

### EXEMPLES

### EXEMPLE 1: Matériels et méthodes

### Procédure générale pour les mesures de tension de surface

Les tensions superficielles ont été mesurées à (25,0 ± 0,1)°C avec un tensiomètre de Krüss K100MK2 à l'aide d'une tige de platine en tant que sonde. Un total de 1,0 ml d'eau a été ajouté à la cuve de mesure. Lorsque la tension de surface est stable (écart type des cinq dernières mesures de 0,2 mN / m), une dilution manuelle est effectuée avec une solution concentrée d'agent tensioactif à sa limite de solubilité tout en maintenant le volume constant.

### Méthodes analytiques

Tous les réactifs et solvants utilisés pour la synthèse sont des produits commerciaux (fournis par SIGMA-ALDRICH et ACROS ORGANICS) qui ont été utilisés sans autre purification. Tous les nouveaux composés obtenus ont été caractérisés par des données spectroscopiques. Les réactions ont été suivies par chromatographie sur couche mince en utilisant un gel de silice sur des plaques d'aluminium (60F254). Les mesures de Résonance Magnétique Nucléaire (RMN) ont été enregistrées sur un spectromètre Bruker DRX 300 ou 400 ou Bruker ALS 300. Les déplacements chimiques sont donnés en référence aux pics centraux du d₆-DMSO ou du CDCI₃ (respectivement 39,5 et 77,0ppm) pour la RMN ¹³C, et (respectivement 2,50 et 7,26 ppm) pour la RMN ¹H. Les constantes de couplages J sont données en Hertz (Hz). Les abréviations doivent se comprendre comme suit: s = singulet, d = doublet, dd = doublet de doublets, t = triplet, q = quadruplet, m = multiplet. Les séparations de chromatographie flash ont été effectuées à l'aide de gel de silice Grace Davisil® LC60A (40-63µ). Les analyses de chromatographie liquide à haute performance ont été effectuées sur colonne de à l'aide d'une colonne C18 (SPHERISORB C18, 5 µm, 250 mm × 20 mm) en utilisant un éluant MeCN - eau (20/80) et une détection par indice de réfraction (RI).

Les spectres de masse ont été acquis en mode ions positifs en utilisant un spectromètre (MicroTOFQ-II, BrukerDaltonics, Bremen) avec une ionisation électrospray (ElectroSprayIonizationESI). Le flux de gaz de pulvérisation est à 0,6 bar et la tension capillaire est de 4,5 kV. Les solutions ont été injectées à 180 µL / h dans un mélange de solvants (méthanol / dichlorométhane / eau 45/40/15). La plage de masse de l'analyse est 50-1000 m / z et l'étalonnage a été effectué avec du formiate de sodium.

Tous les acétals ont été séchés sur du sulfate de magnésium (MgSO₄), et filtrés avant hydrogénolyse.

### Exemple 2: Synthèse des acétals de sorbitane

Tout d'abord, les acétals de sucre ont été préparés par acétalisation ou trans-acétalisation de sucres suivant la procédure décrite dans la demande de brevet FR 3 007 031. Le rapport de 2:1 entre le sucre et l'aldéhyde n'a pas été modifié mais 0,5 équivalent d'un aldéhyde à longue chaîne d'alkyle ou de son acétal a été remplacé par 0,5 équivalent d'un aldéhyde à chaîne d'alkyle courte ou de son acétal afin d'aider à la solubilisation des réactifs (schéma 1). Les produits désirés ont été obtenus sous la forme d'un mélange d'acétals à chaîne courte et à chaîne longue avec des rendements améliorés pour des acétals d'alkyle à longue chaîne, par rapport à la procédure classique.

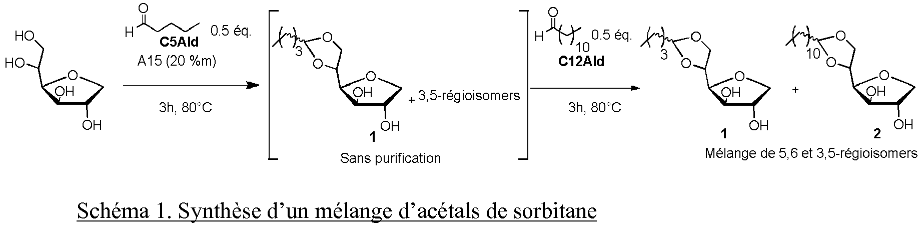

Afin de synthétiser l'acétal du dodecylidène de sorbitane avec un meilleur rendement, du sorbitane a été mis à réagir avec du valéraldéhyde (0,5 équivalent) en présence de 20% en poids d'AMBERLYST® 15 (A15) en tant que catalyseur acide. La réaction peut être effectuée en utilisant du tétrahydrofurane anhydre (1 M) ou de l'éther de méthyle cyclopentyle (1 M) en tant que solvant ou dans des conditions sans solvant. En outre, du sulfate de sodium (1,5 équivalent) a également été ajouté comme agent de déshydratation afin de piéger l'eau formée pendant la réaction d'acétalisation ce qui a induit une augmentation de la conversion de sorbitane. Le sulfate de sodium peut avantageusement être remplacé par des tamis moléculaires ou par une élimination azéotropique de l'eau. Il convient de noter que le sulfate de sodium n'a pas été ajouté à la réaction effectuée dans des conditions sans solvant en raison des difficultés d'agitation. Le mélange réactionnel a été chauffé à 80°C pendant 3 heures, puis 0,5 équivalent de dodécanal a été ajouté et le milieu réactionnel a été agité à 80°C durant 3 heures supplémentaires.

Les résultats sont présentés dans le tableau 1 et comparés à ceux obtenus avec le dodécanal seul.

**Tableau 1. Acétalisation du sorbitane avec un mélange d'aldéhydes à chaîne courte et à chaîne longue^{a}:**

| Entrée | Aldéhyde | Na₂SO₄ (éq.) | Solvant | Conversion **C5Ald**^{b}(%) | Conversion **C12Ald**^{c}(%) | Rendement isolé (**1**+**2**) | Ratio C5:C12^{c} | Rendement **2** |
|---|---|---|---|---|---|---|---|---|
| **1** | C12 | 1.5 | THF | nd | 87 | - | - | 39^{d} |
| **2** | C5/12 | 1.5 | THF | 94 | 86 | 69 | 31:69 | 81^{e} |
| **3** | C5/12 | 1.5 | CPME | 76 | 76 | 62 | 46:54 | 58^{e} |
| **4** | C12 | 0 | sans | nd | 60 (15h) | - | - | 36^{d} |
| **5** | C5/C12 | 0 | sans | 96 | 94 (3h) | 77 | 38:62 | 82^{e} |

| | | | | | | | | |
|---|---|---|---|---|---|---|---|---|
| *^{a}Conditions experimentales: Sorbitane (20 g, 122 mmol), valéraldéhyde (3,2 mL, 31 mmol), AMBERLYST*® *A15 (0,5 g, 20% en poids), 80°C, 3 h, puis le dodécanal (6,9 mL, 31 mmol), 80°C, 3 h. ^{b}Les conversions ont été déterminées par les spectres ¹H-RMN.^{C} Les taux de conversion ont été déterminés par HPLC. ^{d}Rendement isolé. ^{e}Rendement calculé à partir de la masse totale récupérée et le rapport de C5* / *12 tel qu'obtenu par HPLC.* | | | | | | | | |

Après la réaction, le mélange brut a été purifié par chromatographie sur colonne de gel de silice afin d'obtenir un mélange d'acétals C5 / C12. Le rapport entre les acétals de sorbitane en C5 et C12 a ensuite été déterminé par HPLC. Le poids et le rendement en acétal dodecylidène sorbitane (**2**) ont été calculés à partir de ces informations. Le mélange de 3,5- et 5,6-*O*-dodecylidène-1,4-sorbitane (**2**) a été obtenu avec un rendement isolé de 39% (**entrée 1**) en présence de solvant et sans intermédiaire d'acétal tensioactif. Cependant, avec la synthèse du mélange 3,5- et 5,6-pentylidène-1,4-sorbitane (**1**) comme agent tensioactif, l'acétal de sorbitane à longue chaîne alkyle (**2**) souhaité a été isolé à 81% dans le THF (**entrée 2**) et à 58% dans le CPME (**entrée 3**). En outre, le rendement isolé d'acétal de pentylidène et dodécylidene obtenu était de 69% de rendement isolé dans le THF et de 62% dans le CPME. Sans solvant, le phénomène est encore plus important. Dans ce cas, le sulfate de sodium n'a pas été ajouté dans le but de rendre possible l'agitation magnétique. En effet, le rendement isolé en acétal dodecylidène sorbitane est passé de 36% à 82% du fait des caractéristiques tensioactives du pentylidène sorbitane (**entrées 4-5**). Plus précisément, avec l'utilisation de l'acétal pentylidènesorbitane (**1**) *in situ,* le taux de conversion du dodécanal est passé de 60% à 94% et un temps de réaction plus court est nécessaire. Ces résultats indiquent que l'acétal de pentylidène de sorbitane permet la solubilisation des longues chaînes alkyles hydrophobes dans le milieu et présentent des propriétés de « solvo-tensioactif » ou « solvo-surfactant ». L'utilisation de tensioactifs intermédiaires contribue à une meilleure solubilisation des réactifs en diminuant la tension superficielle entre les phases polaires et apolaires. L'APG désiré est ainsi obtenu. En fait, le butanol sert à la fois comme solvant et réactif, pour former le O-butyle glucoside qui va ensuite être miscible avec le FOH pendant la transglycosidation suivante. En outre, cette méthode pourrait être effectuée sur des oligosaccharides dans un seul et même milieu, autrement dit dans un unique réacteur.

### EXEMPLE 3 L'hydrogénolyse des acétals de C5 / C12

L'hydrogénolyse du mélange d'acétals de sorbitane de 3,5- et 5,6-pentylidène (**1**) et de dodecylidène (**2**) a été réalisé en utilisant les conditions déjà décrites par les inventeurs dans une demande de brevet français précédente N°14/01346. Les conditions d'hydrogénolyse optimisées [5 mol% de Pd/ C (5%), 120°C, 30 bar de H₂, CPME (0,1 M), avec une vitesse d'agitation mécanique de 800 tours/min] ont été utilisées pour la synthèse de monoéthers de sorbitane en position 3, 5 et 6 issus des acétals de sucre (**schéma 2**).

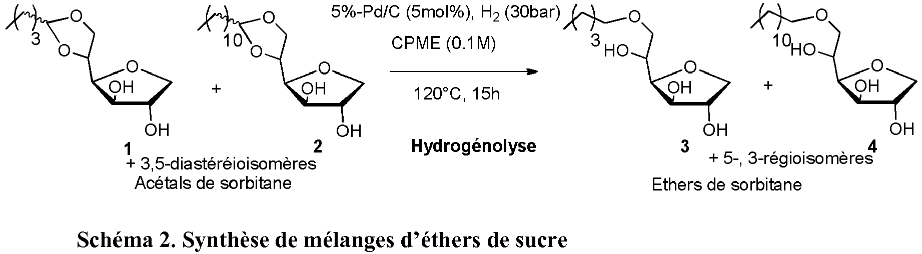

**Tableau 2. L'hydrogénolyse des acétals de sorbitane^{a}:**

| | | | | | | |
|---|---|---|---|---|---|---|
| 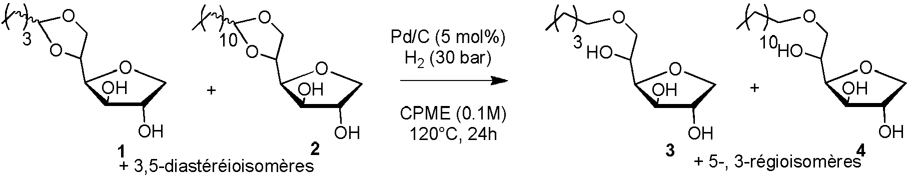 | | | | | | |

| Entrée | Acétal de sucre | Conversion **(1+2)**^{b}(%) | Rendement isolé (**3**+**4**)^{d} | Ratio C5:C12^{c} | Rendement **4** | Sélectivité (%) |
|---|---|---|---|---|---|---|
| **1** | C12 | 97 | - | - | 55^{d} | 57 |
| **2*** | C5/12 | 65 | 18 | 36:64 | 17^{e} | 26 |
| **3** | C5/C12 | >98 | 89 | 48:52 | 68^{e} | 69 |

| | | | | | | |
|---|---|---|---|---|---|---|
| *^{a}Conditions experimentales: sorbitane (20 mmol), Pd* / *C (5% mol, 5% Pd), 120°C, 30 bar H₂, la vitesse d'agitation = 800 rpm* * *agitation magnétique, 15 h. ^{b}Conversions déterminées par les spectres ¹H RMN. ^{d}Rendement isolé. ^{e}Rendements calculés avec le rapport C5* / *12.* | | | | | | |

Les résultats sont résumés dans le tableau 2. Partant du (3,5 + 5,6) - dodécylidène sorbitane (**entrée 1**), l'acétal (**2**) a été totalement transformé. Cependant, dans ces cas, le rendement isolé en (6 + 5 + 3 -) - dodécyle de sorbitane (**4**) a été seulement de 55%. Cependant, partant du précédent mélange d'acétals en C5 / C12 de sorbitane (**entrée 2**), la conversion est plus faible (65%). En effet, sous agitation mécanique, la conversion est totale et la sélectivité atteint 69% au lieu de 57% sans acétal de pentylidène sorbitane pour obtenir le produit désiré avec 68% de rendement.

A partir de ces résultats, les inventeurs ont montré que l'étape limitante de l'acétalisation avec l'aldéhyde alkyle à longue chaîne pourrait être améliorée par l'utilisation d'un acétal d'alkyle à chaîne courte de sorbitane en tant que produit intermédiaire qui joue le rôle de "solvo-tensioactif" par dispersion du réactif hydrophobe dans un milieu polaire. En outre, cette étude a montré que les éthers de sorbitane à longues chaînes alkyle peuvent être synthétisés avec un meilleur rendement à partir du mélange C5 / C12.

### Exemple 4: Propriétés physico-chimiques des agents tensioactifs synthétisés

### Propriétés tensioactives

Plusieurs approches peuvent être utilisées afin d'étudier les propriétés des tensioactifs. Dans un premier temps, des essais de tension de surface ont été réalisées entre l'air et l'eau en vue de déterminer l'évolution des propriétés en fonction de la longueur de la chaîne alkyle et de la tête polaire.

Dans la présente étude, les caractéristiques générales des agents tensioactifs ont été évaluées en mesurant la réduction de la tension superficielle saturée (γₛₐₜ) et la concentration micellaire critique (CMC) du mélange (**3** + **4**) précédemment obtenu dans l'eau à 25°C. Les propriétés tensioactives ont été comparées à celles des composés purs.

### La tension de surface des solutions aqueuses

La tension de surface de solutions aqueuses contenant des concentrations croissantes de chacun des composés a été mesurée par la méthode Du Noüy-Padday à l'aide d'une tige de platine en tant que sonde (méthode décrite dans J.F. Padday, A.R. Pitt, R.M. Pashley, J. Chem. Soc. Faraday. Trans 1, 1975, 71, 1919-1931*).* Les données sont représentées graphiquement dans la **figure 1****,** montrant l'analyse de la tension de surface en fonction de la concentration des dérivés d'éthers de sorbitane (C5EthSorb (losanges noires), C12EthSorb (pastilles noires), C5 / C12EthSorb (triangles noires)). La valeur de la concentration micellaire critique (CMC) est indiquée par une petite flèche pour le C5EthSorb.

Pour tous les composés, la réduction de la tension superficielle de l'eau est observée et la valeur de saturation est atteinte à des concentrations très faibles. Selon les courbes, les concentrations micellaires critiques (CMC) et la tension de surface de saturation sont reprises dans le tableau 3.

**Tableau 3. Concentration minimale hydrotropique (CMH) ou concentration micellaire critique (CMC) et la tension superficielle de l'eau (γₛₐₜ).**

| Produit | Rapport(3c:3b:3a)/(4c:4b:4a)^{a} | CMC (mg/L) | γₛₐₜ (mN/m) |
|---|---|---|---|
| 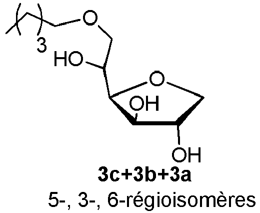 | 26:33:41 | 1779 | 32 |
| **C5EthSorb** | | | |
| **C12EthSorb** | 27:33:40 | 30,2 | 31 |
| **C5/C12EthSorb (48/52)** | 37:16:47/52:38:10 | 29,8 | 30 |

| | | | |
|---|---|---|---|
| *^{a}Rapport de mesures obtenues par HPLC* | | | |

A partir de ces résultats, on peut conclure que pour tous les composés, la réduction de la tension superficielle de l'eau est observée et la valeur de saturation est atteinte à des concentrations très faibles. En outre, il peut être remarqué que le mélange de C5 / C12, dans le rapport 48:52, a donné des propriétés tensioactives similaires à celles de l'éther dodécyle de sorbitane pur (par CMC pour C5 / C12 de 29,8 mg / L et 30,2 mg / L pour C12). Ces résultats démontrent la synergie du mélange C5 / C12. Ainsi, le pentyle de sorbitane (3) permet d'augmenter la solubilité du mélange tout en obtenant des caractéristiques tensioactives similaires à celles du dodécyle de sorbitane pur (**4**). En effet, les dodécyles de sorbitane induisent une diminution de la tension de surface de l'eau même à faible concentration du mélange de tensioactifs. Avec l'éther pentyle de sorbitane, une concentration de 1179 mg / L est nécessaire pour abaisser la tension de surface de 31 mN / m tandis qu'une concentration de 29,8 mg / L du mélange C5 / C12 avec seulement 48% de pentyle de sorbitane suffit pour atteindre cette même tension de surface.

### Exemple 5: Synthèse d'acétals C5 / C12 de glucopyranoside de méthyle

Le 4,6-*O*-dodécylidène-α-D-glucopyranoside de méthyle a été synthétisé comme dans l'exemple 2 avec du valéraldéhyde (0,5 équivalent) en présence de 20% en poids d'AMBERLYST® 15 (A15) en tant que catalyseur acide (voir schéma 3). Comme évoqué précédemment, la réaction peut être effectuée en utilisant du tétrahydrofuranne anhydre (1 M) ou de l'éther de méthyle cyclopentyle (1 M) en tant que solvant ou dans des conditions sans solvant. En outre, le sulfate de sodium (1,5 équivalent) a également été ajouté comme agent de déshydratation. Le mélange réactionnel a été chauffé à 80°C pendant 3 heures, puis le dodécanal (0,5 équivalent) est ajouté et le milieu réactionnel a été agité à 80°C pendant 3 heures supplémentaires.

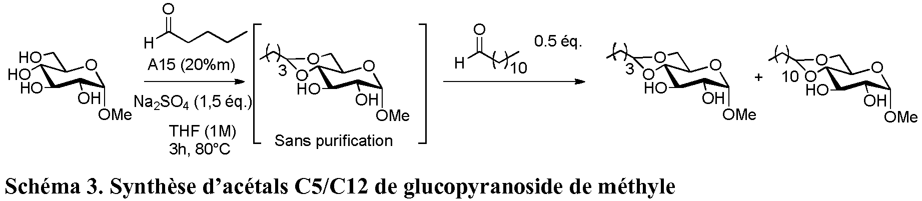

Les résultats sont présentés dans le tableau 4 et comparés à ceux obtenus avec le dodécanal seul.

**Tableau 4. Acétalisation du glucoside de méthyle en utilisant un mélange d'aldéhydes à chaîne courte et à chaîne longue**

| **Entrée** | **Na₂SO₄ (eq.)** | **Aldéhyde** | **Conversion C5Ald^{a}** | **Conversion C12Ald^{b}** | **Rendement isolé** | **Ratio C5:C12^{b}** | **Rendement C12** |
|---|---|---|---|---|---|---|---|
| **1** | 1,5 | C12 | - | 76 | - | | **28** |
| **2** | 0,75+ 0,75 | C5/C12 | 64 | 72 | 41 | 45:55 | **39** |

| | | | | | | | |
|---|---|---|---|---|---|---|---|
| *^{a}Conversions déterminées par ¹H NMR. ^{b}Ratio déterminés par HPLC* | | | | | | | |

Après la réaction, le mélange brut a été purifié par chromatographie sur colonne de gel de silice pour donner un mélange d'acétals C5 / C12 de glucoside de méthyle. Le rapport entre les acétals C5 et C12 de glucoside de méthyle a ensuite été déterminé par HPLC. Le poids et le rendement en acétal dodecylidène de méthyle glucoside ont été calculés à partir de ces informations.

Sans tensioactif intermédiaire, le 4,6-*O*-décanylidène-α-D-glucopyranoside de méthyle, a été obtenu avec un rendement isolé de 28% (**entrée 1**). Cependant, durant la synthèse du 4,6-*O*-pentylidène-α-D-glucopyranoside de méthyle, l'acétal de méthyle α-D-glucopyranoside alkyle à chaîne longue désiré a été isolé dans du THF avec un rendement amélioré d'environ 39% (**entrée 2**). Ces résultats démontrent que le 4,6-*O*-pentylidène α-D-glucopyranoside de méthyle permet la solubilisation de l'alkyle hydrophobe à longue chaîne.

### Exemple 6: Hydrogénolyse des éthers C5 / C12 de glucopyranoside de méthyle

L'hydrogénolyse du mélange de 4,6-*O*-dodécylidène-α-D-glucopyranoside de méthyle a été réalisée en utilisant les conditions déjà décrites dans l'exemple 3 (voir le schéma 4).

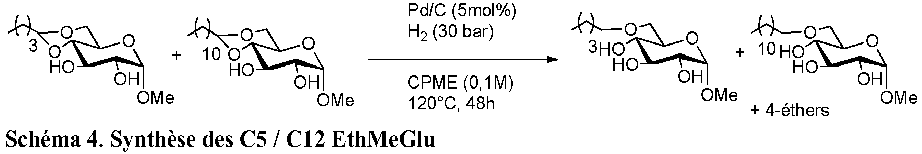

**Tableau 5. L'hydrogénolyse des acétals de glucoside de méthyle^{a}:**

| **Entrée** | **Rapport Acétal C5:C12^{b}** | **Conv.^{c}** | **Rendement isolé C5/C12^{d}** | **Rapport Ethers C5:C12^{b}** | **Rendement calculé C12^{e}** |
|---|---|---|---|---|---|
| **1** | - | 59 | - | - | **51** |
| **2** | 45:55 | 68 | 28 | 46:54 | **28** |

| | | | | | |
|---|---|---|---|---|---|
| *^{a}Conditions experimentales: acétals de glucoside de méthyle (20 mmol), de Pd* / *C (5% mol, 5% de Pd), CPME (200 mL), 120°C, 30 bar de H₂, la vitesse d'agitation est de 800 tours par minute, 15 h. ^{b}Ratio déterminés par HPLC. ^{c}Conversions déterminées par les spectres ¹H RMN. ^{d}Rendement isolé. ^{e}Rendement calculé avec le rapport C5* / *12.* | | | | | |

Les résultats sont résumés dans le tableau 5. A partir du (4,6)-*O*-dodécylidène-α-D-glucopyranoside de méthyle (entrée 1), l'acétal de sucre a été converti à 59%. Cependant, dans ces cas, le rendement calculé en (6 + 4) - *O*-dodécyle-α-D-glucopyranoside de méthyle a été seulement de 51%. Cependant, partant du précédent mélange d'acétals en C5 / C12 de glucoside de méthyle (entrée 2), la conversion est plus élevée (68%). En effet, le dodécyle-α-D-glucopyranoside de méthyle a été obtenu avec seulement 28% de rendement calculé. L'utilisation de l'acétal de glucoside avec une chaîne alkyle courte ne permet pas d'augmenter le rendement en éther de glucoside à longue chaîne alkyle.

### Exemple 7: Propriétés physico-chimiques des agents tensioactifs synthétisés

### Propriétés tensioactives

Les caractéristiques générales des agents tensioactifs ont été évaluées comme dans l'exemple 4 en mesurant la réduction de la tension superficielle saturée (γₛₐₜ) et la concentration micellaire critique (CMC) du mélange (C5 / C12 MeGlu) obtenu précédemment dans l'eau à 25°C. Les propriétés tensioactives ont été comparées à des composés purs.

### La tension de surface des solutions aqueuses

La tension de surface de solutions aqueuses contenant des concentrations croissantes de chacun des composés a été mesurée par la méthode de la plaque à l'aide d'une tige de platine en tant que sonde (méthode Du Nouy-Padday comme décrit dans JF Padday, AR Pitt, RM Pashley, J. Chem. Soc. Faraday. Trans 1, 1975, 71, 1919-1931). Les données sont représentées sur la figure 2, montrant l'analyse de la tension de surface par la concentration des dérivés d'éther de glucoside de méthyle (C5EthMeGlu (étoile noire), C12EthMeGlu (losange noir), C5 / C12EthMeGlu (croix noire)).

Pour tous les composés, la réduction de la tension superficielle de l'eau est observée et la valeur de saturation est atteinte à des concentrations très faibles. Selon les courbes, les concentrations micellaires critiques (CMC) et la tension de surface de saturation sont données dans le tableau 6.

**Tableau 6. Concentration minimale hydrotropique (CMH) ou concentration micellaire critique (CMC) et la tension superficielle de l'eau (γₛₐₜ)**

| Produit | CMC (mg/L) | γₛₐₜ (mN/m) |
|---|---|---|
| 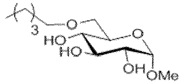 | 8199 | 32 |
| **C5EthMeGlu** | | |
| **C12EthMeGlu** | 4,5 | 28 |
| **C5/C12EthMeGlu (46/54)** | 18,9 | 30 |

A partir de ces résultats et comme indiqué précédemment pour les éthers de sorbitane, on peut conclure que pour tous les composés, une réduction de la tension superficielle de l'eau est observée et la valeur de saturation est atteinte à des concentrations très faibles. En outre, des propriétés tensioactives similaires sont également observées entre le mélange C5 / C12, dans le rapport 46:54 et le dodécyle de α-D-glucopyranoside de méthyle pur (avec une CMC de 18,9 mg / L pour le mélange C5 / C12 et de 4,5 mg/L pour C12). Comme observé précédemment dans l'exemple 4 pour les éthers de sorbitane, ces résultats confirment la synergie du mélange C5 / C12. En effet, le pentyle α-D-glucopyranoside de méthyle joue le rôle de "solvo-tensioactif' par l'amélioration de la solubilité du mélange, et plus particulièrement du dodécyle de α-D-glucopyranoside de méthyle. En outre, à faible concentration, le dodécyle de α-D-glucopyranoside de méthyle diminue la tension superficielle de l'eau. Avec le pentyle α-D-glucopyranoside de méthyle, une concentration de 8199 mg / L est nécessaire pour abaisser la tension de surface de 32 mN / m tandis que seulement 18,9 mg / L du mélange C5 / C12 est nécessaire pour atteindre une tension de surface de 30 mN/m.

## Revendications

1. Procédé d'obtention d'un mélange de monoéther d'alkyle en C4-C8 de saccharide et/ou de dérivé de saccharide et de monoéther d'alkyle en C9-C18 de saccharide et/ou de dérivé de saccharide, ledit dérivé de saccharide étant un alkylglycoside ou saccharide alkylé et/ou saccharide hydrogéné et/ou saccharide déshydraté, le procédé comprenant:
a) une première étape d'acétalisation ou de trans-acétalisation d'un saccharide, de dérivé de saccharide ou de leurs mélanges par un aldéhyde aliphatique en C4-C8 ou l'acétal de celui-ci,
b) une deuxième étape consécutive ou simultanée d'acétalisation ou de trans-acétalisation du produit obtenu en a), du saccharide, du dérivé de saccharide ou de leurs mélanges, par un aldéhyde aliphatique en C9 à C18 ou l'acétal de celui-ci,
c) une étape d'hydrogénolyse catalytique des acétals de saccharide et/ou de dérivé de saccharide obtenus en b), et
d) une étape de récupération d'un mélange de monoéther d'alkyle en C4-C8 de saccharide et/ou de dérivé de saccharide et de monoéther d'alkyle en C9-C18 de saccharide et/ou de dérivé de saccharide.

2. Procédé selon la revendication 1, dans lequel ledit dérivé de saccharide est un anhydrosaccharide de préférence un monoanhydrosaccharide ou un glycoside d'alkyle, de préférence un glycoside d'alkyle en C1-C4.

3. Procédé selon l'une ou l'autre des revendications 1 et 2, dans lequel ledit saccharide est un monosaccharide, un disaccharide ou un trisaccharide.

4. Procédé selon l'une quelconque des revendications 1 à 3, dans lequel ledit saccharide et/ou le dérivé de saccharide comprend de 4 à 7 atomes de carbone, de préférence, ledit saccharide et/ou le dérivé de saccharide est choisi parmi :
- un hexose de préférence choisi dans un groupe constitué par le glucose, le mannose, le galactose, l'allose, l'altrose, le gulose, l'idose ou le talose,
- un hexitan, de préférence, choisi dans un groupe constitué par le 1,4-anhydro-D-sorbitol; 1,5-anhydro-D-sorbitol; 3,6-anhydro-D-sorbitol; 1,4 (3,6) -anhydro-D-mannitol; 1,5-anhydro-D-mannitol; 3,6-anhydro-D-galactitol; 1,5-anhydro-D-galactitol; 1,5-anhydro-D-talitol; et le 2,5-anhydro-L-iditol.

5. Procédé selon l'une quelconque des revendications 1 à 4, dans lequel ledit dérivé de saccharide est un sucre-alcool choisi dans le groupe constitué par l'érythritol, le thréitol, l'arabitol, le xylitol, le ribitol, le mannitol, le sorbitol, le galactitol, l'iditol, le volémitol, l'isomalt, le maltitol, le lactitol, le maltotriitol, le maltotétraitol et le polyglycitol, de préférence lorsque ledit saccharide est un sucre-alcool, le procédé comprend en outre une étape de déshydratation, avant ladite étape a) de première acétalisation ou trans-acétalisation.

6. Procédé selon l'une quelconque des revendications 1 à 5, dans lequel ledit dérivé de saccharide est un glycoside d'alkyle choisi parmi un groupe constitué du méthyle glucoside, du glucoside d'éthyle, du glucoside de propyle, du glucoside de butyle, du xyloside de méthyle, du xyloside d'éthyle, du xyloside de propyle, du xyloside de butyle, du mannoside de méthyle, du mannoside d'éthyle, du mannosidedepropyle, du mannoside de butyle, du galactoside de méthyle, du galactoside d'éthyle, du galactoside de propyle et du galactoside de butyle.

7. Procédé selon l'une quelconque des revendications 1 à 6, dans lequel l'aldéhyde aliphatique en C4-C8 est un aldéhyde aliphatique en C5 ou l'acétal de celui-ci et/ou l'aldéhyde aliphatique en C9 à C18 ou l'acétal de celui-ci est un aldéhyde aliphatique en C12 ou l'acétal de celui-ci.

8. Procédé selon l'une quelconque des revendications 1 à 7, dans lequel le rapport molaire aldéhyde aliphatique en C4-C8 ou en C9-C18 ou leur acétal : saccharide, le dérivé de saccharide ou leurs mélanges est compris entre 5:1 et 1:5.

9. Procédé selon l'une quelconque des revendications 1 à 8, dans lequel la première et/ou la deuxième étape d'acétalisation ou de trans-acétalisation est effectuée en présence d'un catalyseur acide, de préférence la première et/ou la deuxième étape d'acétalisation ou de trans-acétalisation est réalisée dans des conditions sans solvant ou en présence d'un solvant polaire.

10. Procédé selon l'une quelconque des revendications 1 à 9, dans lequel l'hydrogénolyse est effectuée à une température comprise entre 80 et 140°C et/ou à une pression comprise entre 15 et 50 bar, de préférence en présence d'un catalyseur à base de métaux précieux, de métaux commun préférentiellement dans le groupe des métaux ferreux.

11. Composition comprenant un mélange d'isomères de position de monoéther d'alkyle en C4-C8 de saccharide ou de dérivé de saccharide et d'isomères de position de monoéther d'alkyle en C9-C18 de saccharide ou de dérivé de saccharide, dans lequel le dérivé de saccharide est un saccharide alkylé et/ou hydrogéné et/ou déshydraté, et le saccharide est un hexose.

12. Composition selon la revendication 11, dans laquelle le groupement alkyle en C4-C8 est un alkyle en C5 et le groupement alkyle en C9-C18 est un alkyle en C12 préférentiellement, le dérivé de saccharide est choisi parmi le monoanhydro sorbitol ou le glucoside d'alkyle plus préférentiellement le dérivé de saccharide est le glucoside de méthyle.

13. Composition selon la revendication 12, dans laquelle le rapport monoéther d'alkyle en C5 de saccharide ou de dérivé de saccharide / monoéther d'alkyle en C12 de saccharide ou de dérivé de saccharide est compris entre 5:95 et 95:5.

14. Utilisation du mélange obtenu selon le procédé selon l'une quelconque des revendications 1 à 10 comme un agent tensioactif, de préférence ledit agent tensioactif est choisi parmi un détergent, un émulsifiant, un stabilisateur d'émulsion, un agent moussant, un stabilisant de mousse, un stabilisant de liposome, un dispersant et un agent mouillant.

15. Utilisation d'une composition comprenant un mélange de monoéther d'alkyle en C4-C8 de saccharide et/ou de dérivé de saccharide et de monoéther d'alkyle en C9-C18 de saccharide et/ou de dérivé de saccharide selon les revendications 11 à 13 en tant qu'agent tensioactif, de préférence ledit agent tensioactif est choisi parmi un détergent, un émulsifiant, un stabilisateur d'émulsion, un agent moussant, un stabilisant de mousse, un stabilisant de liposome, un dispersant et un agent mouillant.

## Patentansprüche

1. Verfahren zum Erhalt eines Gemischs von Saccharid- und/oder Saccharidderivat-C4-C8-alkylmonoether und Saccharid- und/oder Saccharidderivat-C9-C18-alkyl-monoether, wobei sich bei dem Saccharidderivat um ein Alkylglycosid oder alkyliertes Saccharid und/oder hydriertes Saccharid und/oder dehydratisiertes Saccharid handelt, wobei das Verfahren Folgendes umfasst:
a) einen ersten Schritt der Acetalisierung oder Umacetalisierung eines Saccharids, eines Saccharidderivats oder von Gemischen davon mit einem aliphatischen C4-C8-Aldehyd oder dem Acetal davon,
b) einen nachfolgenden oder gleichzeitigen zweiten Schritt der Acetalisierung oder Umacetalisierung des in a) erhaltenen Produkts, des Saccharids, des Saccharidderivats oder von Gemischen davon mit einem aliphatischen C9-C18-Aldehyd oder dem Acetal davon,
c) einen Schritt der katalytischen Hydrogenolyse der in b) erhaltenen Saccharid- und/oder Saccharidderivatacetale und
d) einen Schritt des Gewinnens eines Gemischs von Saccharid- und/oder Saccharidderivat-C4-C8-alkyl-monoether und Saccharid- und/oder Saccharidderivat-C9-C18-alkylmonoether.

2. Verfahren nach Anspruch 1, bei dem es sich bei dem Saccharidderivat um ein Anhydrosaccharid, vorzugsweise ein Monoanhydrosaccharid oder ein Alkylglycosid, vorzugsweise ein C1-C4-Alkylglycosid, handelt.

3. Verfahren nach einem der Ansprüche 1 und 2, bei dem es sich bei dem Saccharid um ein Monosaccharid, ein Disaccharid oder ein Trisaccharid handelt.

4. Verfahren nach einem der Ansprüche 1 bis 3, bei dem das Saccharid und/oder Saccharidderivat 4 bis 7 Kohlenstoffatomen umfasst, vorzugsweise das Saccharid und/oder Saccharidderivat aus:
- einer Hexose, vorzugsweise aus der Gruppe bestehend aus Glucose, Mannose, Galactose, Allose, Altrose, Gulose, Idose oder Talose,
- einem Hexitan, vorzugsweise aus der Gruppe bestehend aus 1,4-Anhydro-D-sorbitol; 1,5-Anhydro-D-sorbitol; 3,6-Anhydro-D-sorbitol; 1,4(3,6)-Anhydro-D-mannitol; 1,5-Anhydro-D-mannitol; 3,6-Anhydro-D-galactitol; 1,5-Anhydro-D-galactitol; 1,5-Anhydro-D-talitol und 2,5-Anhydro-L-iditol,
ausgewählt wird.

5. Verfahren nach einem der Ansprüche 1 bis 4, bei dem es sich bei dem Saccharidderivat um einen Zuckeralkohol aus der Gruppe bestehend aus Erythritol, Threitol, Arabitol, Xylitol, Ribitol, Mannitol, Sorbitol, Galactitol, Iditol, Volemitol, Isomalt, Maltitol, Lactitol, Maltotriitol, Maltotetraitol und Polyglycitol handelt, vorzugsweise das Verfahren dann, wenn es sich bei dem Saccharid um einen Zuckeralkohol handelt, außerdem einen Dehydratisierungsschritt vor dem Schritt a) der ersten Acetalisierung oder Umacetalisierung umfasst.

6. Verfahren nach einem der Ansprüche 1 bis 5, bei dem es sich bei dem Saccharidderivat um ein Alkylglycosid aus der Gruppe bestehend aus Methylglucosid, Ethylglucosid, Propylglucosid, Butylglucosid, Methylxylosid, Ethylxylosid, Propylxylosid, Butylxylosid, Methylmannosid, Ethylmannosid, Propylmannosid, Butylmannosid, Methylgalactosid, Ethylgalactosid, Propylgalactosid und Butylgalactosid handelt.

7. Verfahren nach einem der Ansprüche 1 bis 6, bei dem es sich bei dem aliphatischen C4-C8-Aldehyd um einen aliphatischen C5-Aldehyd oder das Acetal davon handelt und/oder es sich bei dem aliphatischen C9-C18-Aldehyd oder dem Acetal davon um einen aliphatischen C12-Aldehyd oder das Acetal davon handelt.

8. Verfahren nach einem der Ansprüche 1 bis 7, bei dem das Molverhältnis von aliphatischem C4-C8- oder C9-C18-Aldehyd oder dem Acetal davon zu Saccharid, Saccharidderivat oder Gemischen davon zwischen 5:1 und 1:5 liegt.

9. Verfahren nach einem der Ansprüche 1 bis 8, bei dem der erste und/oder der zweite Acetalisierungs- oder Umacetalisierungsschritt in Gegenwart eines sauren Katalysators durchgeführt wird, vorzugsweise der erste und/oder der zweite Acetalisierungs- oder Umacetalisierungsschritt unter Bedingungen ohne Lösungsmittel oder in Gegenwart eines polaren Lösungsmittels durchgeführt wird.

10. Verfahren nach einem der Ansprüche 1 bis 9, bei dem die Hydrogenolyse bei einer Temperatur zwischen 80 und 140 °C und/oder bei einem Druck zwischen 15 und 50 bar, vorzugsweise in Gegenwart eines Katalysators auf Basis von Edelmetallen oder Unedelmetallen, vorzugsweise aus der Gruppe der Eisenmetalle, durchgeführt wird.

11. Zusammensetzung, umfassend ein Gemisch von Stellungsisomeren von Saccharid- oder Saccharidderivat-C4-C8-alkylmonoether und Stellungsisomeren von Saccharid- oder Saccharidderivat-C9-C18-alkyl-monoether, wobei sich bei dem Saccharidderivat um ein Alkylglycosid oder alkyliertes Saccharid und/oder hydriertes Saccharid und/oder dehydratisiertes Saccharid handelt und es sich bei dem Saccharid um eine Hexose handelt.

12. Zusammensetzung nach Anspruch 11, wobei es sich bei der C4-C8-Alkylgruppe um ein C5-Alkyl handelt und es sich bei der C9-C18-Alkylgruppe um ein C12-Alkyl handelt, vorzugsweise das Saccharidderivat aus Monoanhydrosorbitol oder Alkylglucosid ausgewählt ist, weiter bevorzugt es sich bei dem Saccharidderivat um Methylglucosid handelt.

13. Zusammensetzung nach Anspruch 12, wobei das Verhältnis von Saccharid- oder Saccharidderivat-C5-alkylmonoether zu Saccharid- oder Saccharidderivat-C12-alkylmonoether zwischen 5:95 und 95:5 liegt.

14. Verwendung eines gemäß dem Verfahren nach einem der Ansprüche 1 bis 10 erhaltenen Gemischs als grenzflächenaktives Mittel, vorzugsweise wobei das grenzflächenaktive Mittel aus einem Detergens, einem Emulgator, einem Emulsionsstabilisator, einem Schaummittel, einem Schaumstabilisator, einem Liposomenstabilisator, einem Dispergiermittel und einem Netzmittel ausgewählt ist.

15. Verwendung einer Zusammensetzung, die ein Gemisch von Saccharid- und/oder Saccharidderivat-C4-C8-alkylmonoether und Saccharid- und/oder Saccharidderivat-C9-C18-alkylmonoether umfasst, nach einem der Ansprüche 11 bis 13, als grenzflächenaktives Mittel, vorzugsweise wobei das grenzflächenaktive Mittel aus einem Detergens, einem Emulgator, einem Emulsionsstabilisator, einem Schaummittel, einem Schaumstabilisator, einem Liposomenstabilisator, einem Dispergiermittel und einem Netzmittel ausgewählt ist.

## Claims

1. Process for obtaining a mixture of a C₄-C₈ alkyl monoether of disaccharide and/or of saccharide derivative and of C₉-C₁₈ alkyl monoether of saccharide and/or of saccharide derivative, said saccharide derivative being an alkyl glycoside or alkylated saccharide and/or hydrogenated saccharide and/or dehydrated saccharide, the process comprising:
a) a first step of acetalization or of trans-acetalization of a saccharide, of a saccharide derivative or of mixtures thereof by a C₄-C₈ aliphatic aldehyde or the acetal thereof,
b) a consecutive or simultaneous second step of acetalization or of trans-acetalization of the product obtained in a), of the saccharide, of the saccharide derivative or of mixtures thereof, by a C₉ to C₁₈ aliphatic aldehyde or the acetal thereof,
c) a step of catalytic hydrogenolysis of the saccharide acetals and/or saccharide derivative acetals obtained in b), and
d) a step of recovery of a mixture of C₄-C₈ alkyl monoether of saccharide and/or of saccharide derivative and of C₉-C₁₈ alkyl monoether of saccharide and/or of saccharide derivative.

2. Process according to Claim 1, in which said saccharide derivative is an anhydrosaccharide, preferably a monoanhydrosaccharide, or an alkyl glycoside, preferably a C₁-C₄ alkyl glycoside.

3. Process according to either of Claims 1 and 2, in which said saccharide is a monosaccharide, a disaccharide or a trisaccharide.

4. Process according to any one of Claims 1 to 3, in which said saccharide and/or the saccharide derivative comprises from 4 to 7 carbon atoms, preferably said saccharide and/or the saccharide derivative is chosen from:
- a hexose, preferably chosen from a group consisting of glucose, mannose, galactose, allose, altrose, gulose, idose or talose,
- a hexitan, preferably chosen from the group consisting of 1,4-anhydro-D-sorbitol; 1,5-anhydro-D-sorbitol; 3,6-anhydro-D-sorbitol; 1,4(3,6)-anhydro-D-mannitol; 1,5-anhydro-D-mannitol; 3,6-anhydro-D-galactitol; 1,5-anhydro-D-galactitol; 1,5-anhydro-D-talitol; and 2,5-anhydro-L-iditol.

5. Process according to any one of Claims 1 to 4, in which said saccharide derivative is a sugar alcohol chosen from the group consisting of erythritol, threitol, arabitol, xylitol, ribitol, mannitol, sorbitol, galactitol, iditol, volemitol, isomalt, maltitol, lactitol, maltotriitol, maltotetraitol and polyglycitol; preferably, when said saccharide is a sugar alcohol, the process also comprises a dehydration step, before said step a) of first acetalization or trans-acetalization.

6. Process according to any one of Claims 1 to 5, in which said saccharide derivative is an alkyl glycoside chosen from a group consisting of methyl glucoside, ethyl glucoside, propyl glucoside, butyl glucoside, methyl xyloside, ethyl xyloside, propyl xyloside, butyl xyloside, methyl mannoside, ethyl mannoside, propyl mannoside, butyl mannoside, methyl galactoside, ethyl galactoside, propyl galactoside and butyl galactoside.

7. Process according to any one of Claims 1 to 6, in which the C₄-C₈ aliphatic aldehyde is a C₅ aliphatic aldehyde or the acetal thereof and/or the C₉ to C₁₈ aliphatic aldehyde or the acetal thereof is a C₁₂ aliphatic aldehyde or the acetal thereof.

8. Process according to any one of Claims 1 to 7, in which the C₄-C₈ or C₉-C₁₈ aliphatic aldehyde or acetal thereof: saccharide, saccharide derivative or mixtures thereof molar ratio is between 5:1 and 1:5.

9. Process according to any one of Claims 1 to 8, in which the first and/or the second acetalization or trans-acetalization step is carried out in the presence of an acid catalyst, preferably the first and/or the second acetalization or trans-acetalization step is carried out under solvent-free conditions or in the presence of a polar solvent.

10. Process according to any one of Claims 1 to 9, in which the hydrogenolysis is carried out at a temperature of between 80 and 140°C and/or at a pressure of between 15 and 50 bar, preferably in the presence of a catalyst based on precious metals, on common metals preferentially in the group of ferrous metals.

11. Composition comprising a mixture of positional isomers of C₄-C₈ alkyl monoether of saccharide or of saccharide derivative and of positional isomers of C₉-C₁₈ alkyl monoether of saccharide or of saccharide derivative, in which the saccharide derivative is an alkylated and/or hydrogenated and/or dehydrated saccharide, and the saccharide is a hexose.

12. Composition according to Claim 11, in which the C₄-C₈ alkyl group is a C₅ alkyl and the C₉-C₁₈ alkyl group is a C₁₂ alkyl; preferentially, the saccharide derivative is chosen from monoanhydrosorbitol and alkyl glucoside, more preferentially the saccharide derivative is methyl glucoside.

13. Composition according to Claim 12, in which the C₅ alkyl monoether of saccharide or of saccharide derivative / C₁₂ alkyl monoether of saccharide or of saccharide derivative ratio is between 5:95 and 95:5.

14. Use of the mixture obtained according to the process according to any one of Claims 1 to 10, as a surfactant; preferably, said surfactant is chosen from a detergent, an emulsifier, an emulsion stabilizer, a foaming agent, a foam stabilizer, a liposome stabilizer, a dispersant and a wetting agent.

15. Use of a composition comprising a mixture of C₄-C₈ alkyl monoether of saccharide and/or of saccharide derivative and of C₉-C₁₈ alkyl monoether of saccharide and/or saccharide derivative according to Claims 11 to 13, as a surfactant; preferably, said surfactant is chosen from a detergent, an emulsifier, an emulsion stabilizer, a foaming agent, a foam stabilizer, a liposome stabilizer, a dispersant and a wetting agent.
